# EUROPEAN PATENT APPLICATION

(11) **EP 2 491 928 A2**
(43) Date of publication of application: **29.08.2012**
(21) Application number: 12168947.5
(22) Date of filing: 17.10.2008
(51) Int. Cl.: A61K 31/416, A61K 31/593, A61K 31/7076, A61K 33/18, A61K 38/27, A61P 17/00, A61K 31/56, A61K 45/06

(54) **Therapeutic agent for vitiligo and method of accelerating pigmentation**

(30) Priority: 19.10.2007 JP 2007273181; 03.04.2008 JP 2008097207
(62) Divisional of application: 08838612.3
(71) Applicant: The University of Tokyo, Bunkyo-ku, Tokyo 113-8654 (JP); MARUHO Co., Ltd., Osaka-shi, Osaka 531-0071 (JP)
(72) Inventor: Takami, Kunihiko, Tokyo, Tokyo 113-8654 (JP); Kikuchi, Kanako, Tokyo, Tokyo 113-8654 (JP); Komine, Mayumi, Shimotsuke-shi, Tochigi 329-0431 (JP); Fujimoto, Seiki, Kyoto-shi, Kyoto 600-8815 (JP)
(74) Representative: Pfenning, Meinig & Partner GbR

(57) **Abstract**

Provided are a pharmaceutical product capable of treating vitiligo by thickening the epidermis at a vitiligo-affected site, which has been thinned, to thereby accelerate pigmentation, and a method of accelerating pigmentation by thickening the epidermis.

Vitiligo can be treated by using a compound having an epidermis thickening action, for example, a therapeutic agent for a skin ulcer which is used in the treatment of burn ulcer, crural ulcer, diabetic ulcer or post-operative ulcer, or a therapeutic agent for bedsore.

## Description

### TECHNICAL FIELD

The present invention relates to a pharmaceutical product which can treat vitiligo by thickening the epidermis to thereby accelerate pigmentation, and a method of accelerating pigmentation by thickening the epidermis.

### BACKGROUND ART

Vitiligo vulgaris is an acquired disease which occurs in an unexpected fashion, and appears as large and small, round-shaped or irregular-shaped, almost completely depigmented macules, in which the boundaries formed by the lack of pigment (melanin) at a portion of the epidermis are clear and distinct. It occurs as a result of destruction or reduction of melanocytes, or a reduction or an interruption of melanin generation by melanocytes.

Vitiligo vulgaris is not a single disease, and is believed to be caused by many kinds of factors. In regard to the etiology, there have been suggested a theory of autoimmunity, which is based on the fact that there are many autoimmune diseases such as hyperthyroidism, as the complications of vitiligo vulgaris; a theory of auto-destruction, which speculates that since some of the intermediate metabolites of melanin synthesis are toxic to melanocytes, melanocytes are destroyed by these; a neural theory based on the fact that in segmental type vitiligo vulgaris, depigmented macules occur along the extension of the nerves; and the like. However, the details are obscure.

Vitiligo vulgaris often occurs at exposed sites such as the face and limbs, and thus patients wish to treat the disease from the viewpoint of QOL (quality of life). However, since the disease is an intractable cutaneous disease, there is not available any effective treatment method.

In many cases, steroid preparations for external use are used as medicaments for the treatment of vitiligo vulgaris, but their therapeutic effects are not satisfactory. Activated vitamin D₃-containing preparations for external use are also used as other medicaments. Furthermore, phototherapies called the PUVA therapy or the narrow-band UVB therapy are in use. Not only the treatment outcomes of these phototherapies are excellent as compared with the steroid preparations for external use, but also the phototherapies tend to be accompanied by more satisfactory outcomes as the frequency of irradiation is higher. However, in order for a patient to receive a treatment according to a phototherapy, the patient must regularly visit a medical institution which has introduced an apparatus, and this imposes a burden on the patient. Therefore, it is desired to develop medicaments for the treatment.

The survival of melanocytes or pigment production is affected by various cytokines secreted from the neighboring cells, including keratinocytes, or by hormones (stem cell growth factor (SCF), α-melanocyte stimulating hormone (α-MSH), endothelin-1, granulocyte macrophage colony stimulating factor (GM-CSF), prostaglandin E2, or the like). In vitiligo-affected sites, the keratinocytes have a possibility that these factors have not been produced satisfactorily. In fact, a decrease in the SCF has been identified in vitiligo-affected sites.

Furthermore, some reports describe that keratinocyte apoptosis and vitiligo are deeply related since the percentage of the occurrence of keratinocyte apoptosis in vitiligo-affected sites is higher as compared with normal sites, and if apoptosis is suppressed, pigmentation occurs at the vitiligo-affected sites (Patent Document 1, Non-Patent Document 1).

Skin diseases are often accompanied by thickening of the epidermis. For examples, at the lesion sites of skin diseases such as chronic eczema, chronic dermatitis (atopic dermatitis), seborrheic keratosis, lichen planus, psoriasis, and spinocellular carcinoma, thickening of the epidermis is observed.

The relationships between apoptosis and skin diseases have been known for long, and for example, in psoriasis or spinocellular carcinoma, keratinocytes which have undergone apoptosis are hardly observed, but rather those factors operating in a suppressive manner on the induction of apoptosis are heavily expressed. On the other hand, in regard to skin diseases such as chronic eczema, chronic dermatitis (atopic dermatitis), seborrheic keratosis and lichen planus, it is known that keratinocytes which have undergone apoptosis are observed in the epidermis of these lesion sites. As can be seen from this, there is no direct relationship between keratinocyte apoptosis and the thickness of the epidermis.

Concerning the therapeutic agents for vitiligo vulgaris, various attempts have been made to accelerate the production of melanin by directly activating melanocytes, but the attempts have not yet been put to practical use.

Therapeutic agents for skin ulcers such as burn ulcer, crural ulcer, diabetic ulcer and post-operative ulcer, and therapeutic agents for bedsore are medications generally used in the field of medicine, but these agents have not been used as the therapeutic agents for vitiligo.
Patent Document 1: WO 2006/088310
Non-Patent Document 1: J. Invest. Dermatol., 124, 976-983, 2005

### DISCLOSURE OF THE INVENTION

### PROBLEM TO BE SOLVED BY THE INVENTION

The present invention provides a pharmaceutical product capable of treating vitiligo by thickening the epidermis at a vitiligo-affected site to thereby accelerate pigmentation, and a method of accelerating pigmentation by thickening the epidermis.

### MEANS FOR SOLVING THE PROBLEM

The inventors of the present invention observed in a histological manner the state of skin at the vitiligo-affected sites of vitiligo vulgaris patients, and obtained a finding that the state of skin is different from the state of skin at normal sites.

That is, in the skin at the vitiligo-affected sites, the epidermis was obviously thinning as compared with the normal sites. Furthermore, there was recognized a phenomenon in which epidermal processes are flattened and a loss of melanocytes is recognized in the epidermis at the vitiligo-affected sites, so that the epidermis is obviously different from normal epidermis. However, in the vitiligo-affected sites where pigmentation was recognized as a result of a treatment involving narrow-band UVB, the epidermis was thickened, the epidermal processes became normal, and the appearance of melanocytes was recognized.

From this observation, the inventors found that if the epidermis at vitiligo-affected sites that is thinning could be thickened, the epidermal processes would become normal and melanocytes would appear to induce pigmentation, thus completing the present invention.

That is, according to a first embodiment of the present invention, there is provided a therapeutic agent for vitiligo containing a compound having an epidermis thickening action, or a pharmacologically acceptable salt thereof, as an active pharmaceutical ingredient.

According to a second embodiment of the present invention, there is provided a therapeutic agent for vitiligo, in which the compound having an epidermis thickening action is selected from therapeutic agents for skin ulcers which are used in the treatment of skin ulcers such as burn ulcer, crural ulcer, diabetic ulcer and post-operative ulcer, and therapeutic agents for bedsore.

According to a third embodiment of the present invention, there is provided a therapeutic agent for vitiligo, in which the compound having an epidermis thickening action is a compound represented by the formula (I) : 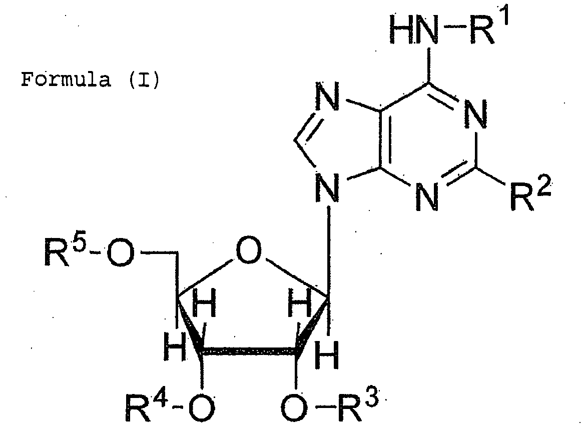 wherein R¹, R² and R³, which may be identical or different, are each a hydrogen atom, substituted or unsubstituted lower alkoxy, or substituted or unsubstituted lower alkanoyl; and
R⁴ and R⁵, which may be identical or different, are each a hydrogen atom, or substituted or unsubstituted lower alkoxy, or R⁴ and R⁵ may be joined together to form a phosphoric acid ester,
or a pharmacologically acceptable salt thereof, and preferably the compound is N,2'-O-dibutyryladenosine-3',5'-phosphoric acid, 2-[2-(4-chlorophenyl)ethoxy]adenosine, or a pharmacologically acceptable salt thereof.

According to a fourth embodiment of the present invention, there is provided a therapeutic agent for vitiligo, in which the compound having an epidermis thickening action is a compound represented by the formula (II) : 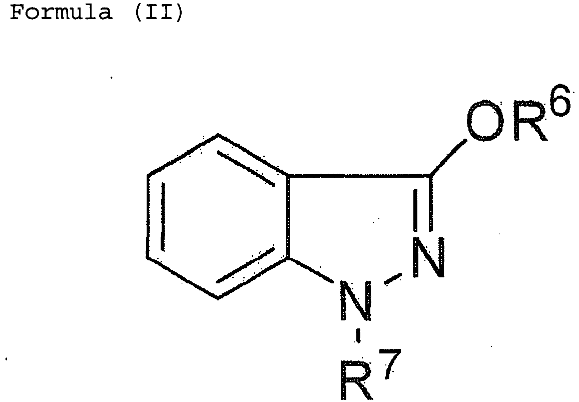 wherein R⁶ and R⁷, which may be identical or different, each represent a hydrogen atom, lower alkyl, carbonyl-lower alkyl, or substituted or unsubstituted aryl-lower alkyl,
or a pharmacologically acceptable salt thereof, and preferably, the compound is [(1-benzyl-1H-indazol-3-yl)oxy]acetic acid or a pharmacologically acceptable salt thereof.

According to a fifth embodiment of the present invention, there is provided a therapeutic agent for vitiligo, in which the compound having an epidermis thickening action is selected from fibroblast growth factors (FGFs) such as acidic fibroblast growth factors (aFGF, FGF-1) and basic fibroblast growth factors (bFGF, FGF-2); and growth factors such as epidermal growth factors (EGFs), vascular endothelial growth factors (VEGFs), keratinocyte growth factors (KGFs) and platelet-derived growth factors (PDGFs), and preferably the compound is a recombinant type basic fibroblast growth factor.

According to a sixth embodiment of the present invention, there is provided a therapeutic agent for vitiligo, in which the compound having an epidermis thickening action is (±)-(2E,4E,6E,8E)-3,7-dimethyl-9-(2,6,6-trimethyl-1-cyclohexen-1-yl)-2,4,6,8-nonatetraenoic acid 3,4-dihydro-2,5,7,8-tetramethyl-2-(4,8,12-trimethyltridecyl)-2H-1-benzopyran-6-yl ester, iodine, or povidone-iodine, or a pharmacologically acceptable salt thereof.

The therapeutic agent for vitiligo can also be used as a pigmentation accelerating agent containing the compound having an epidermis thickening action of the present invention, or a pharmacologically acceptable salt thereof, as an active pharmaceutical ingredient.

Furthermore, the present invention can provide a method of accelerating pigmentation or treating vitiligo by thickening the epidermis. Additionally, the present invention can provide a method of accelerating pigmentation or treating vitiligo by using a compound having an epidermis thickening action or a pharmacologically acceptable salt thereof in combination with a phototherapy, or using a compound having an epidermis thickening action or a pharmacologically acceptable salt thereof in combination with a steroid preparation for external use or an activated vitamin D₃-containing preparation for external use.

### EFFECT OF THE INVENTION

By providing a medicament capable of accelerating pigmentation, the treatment of vitiligo can be carried out without frequently visiting a medical institution for a treatment based on a phototherapy, and therefore the burden on patients can be reduced.

Furthermore, since it is possible to use the present invention in combination with a phototherapy involving narrow-band UVB or the like, a steroid preparation for external use or an activated vitamin D₃-containing preparation for external use and so on, synergistic effects can be expected due to the combination therapy with these, and pigmentation can be further accelerated.

### BRIEF DESCRIPTION OF THE DRAWINGS

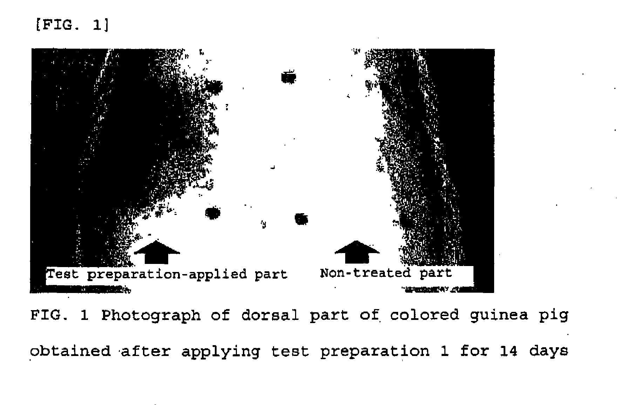

FIG. 1 is a photograph of the dorsal part of a colored guinea pig, obtained after applying a test preparation 1 for 14 days.

### BEST MODE(S) FOR CARRYING OUT THE INVENTION

The lower alkyl may be linear or branched alkyl having 1 to 6 carbon atoms, and specific examples thereof include methyl, ethyl, propyl, isopropyl, butyl, isobutyl, sec-butyl, tert-butyl, pentyl, isopentyl, neopentyl, hexyl, and the like.

The lower alkoxy may be linear or branched alkoxy having 1 to 6 carbon atoms, and specific examples thereof include methoxy, ethoxy, propoxy, isopropoxy, butoxy, sec-butoxy, tert-butoxy, pentoxy, isopentoxy, neopentoxy, hexoxy, and the like.

The aryl may be aryl having 6 to 14 carbon atoms, and specific examples thereof include phenyl, naphthyl, anthryl, and the like.

The lower alkanoyl may be linear or branched alkanoyl having 1 to 6 carbon atoms, and specific examples thereof include acetyl, propyl, butyryl, isobutyryl, pentanoyl, hexanoyl, and the like.

The aryl moiety in the aryl-lower alkyl has the same meaning as that of the aryl described above, and the lower alkyl moiety has the same meaning as that of the lower alkyl described above. Specific examples of the aryl-lower alkyl include benzyl, phenethyl, phenylpropyl, 2-phenylpropyl, phenylbutyl, naphthalenylmethyl, naphthalenylethyl, naphthalenylpropyl, anthracenylmethyl, anthracenylethyl, anthracenylpropyl, and the like.

The lower alkyl moiety in the carbonyl-lower alkyl has the same meaning as that of the lower alkyl described above, and specific examples of the carbonyl-lower alkyl include carbonylmethyl, carbonylethyl, carbonylpropyl, 2-carbonylpropyl, carbonylbutyl, and the like.

The pharmacologically acceptable salt of the compound having an epidermis thickening action that can be used in the present invention may be a conventionally known salt relevant to a basic group such as an amino group or an acidic group such as a hydroxyl group or a carboxyl group. Examples of the salt relevant to a basic group include salts with mineral acids such as hydrochloric acid, hydrobromic acid and sulfuric acid; salts with organic carboxylic acids such as tartaric acid, formic acid, fumaric acid, maleic acid, malic acid and citric acid; and salts with sulfonic acids such as methanesulfonic acid, benzenesulfonic acid, p-toluenesulfonic acid, mesitylenesulfonic acid and naphthalenesulfonic acid. Examples of the salt relevant to an acidic group include salts with alkali metals such as sodium and potassium; salts with alkaline earth metals such as calcium and magnesium; ammonium salts; salts with nitrogen-containing organic bases, such as amino acids such as lysine, arginine and ornithine, trimethylamine, triethylamine, tributylamine, pyridine, N,N-dimethylaniline, N-methylpiperidine, N-methylmorpholine, diethylamine and dicyclohexylamine; and the like.

These compounds can be synthesized according to conventional methods. There are also compounds which are available as pharmaceutical products or reagents. Furthermore, an adenosinic acid derivative such as 2-[2-(4-chlorophenyl)ethoxy]adenosine can be synthesized by the method described in WO 2003/035662.

Furthermore, the various growth factors may be of naturally occurring type, or may be of recombinant type. In regard to the origin, the growth factors may be derived from human being, or may be derived from animals other than human being.

A recombinant type basic fibroblast growth factor is available as Trafermin, a recombinant type epidermal growth factor is available as Palifermin, and a recombinant type vascular endothelial growth factor is available as Telbermin.

The therapeutic agent for vitiligo of the present invention is characterized by containing a medicament having an epidermis thickening action. The therapeutic agent can contain a carrier, an excipient, a colorant, a lubricant, a binding agent, a disintegrant, a coating agent, a base, an emulsifier, a thickener, a stabilizer, a preservative, a solvent, a solubilizing agent, a suspending agent, a buffering agent or the like, which are physiologically or pharmaceutically acceptable and conventionally usable, as long as these agents do not impair the effects of the present invention.

The therapeutic agent for vitiligo of the present invention can be used as a preparation for external use intended for the direct use at the vitiligo-affected sites, and examples of the dosage form thereof include an ointment, a cream, a lotion, a gel, an aerosol, an adhesive patch, and the like. Furthermore, the therapeutic agent can also be used as an injectable preparation or an oral preparation in terms of systemic administration, and examples of the dosage form include an aqueous injectable preparation, an oily injectable preparation, an emulsified injectable preparation, a tablet, a capsule, a granule, a powder, a liquid preparation, and the like. These preparations can be produced by known methods, for example, the methods described in the Japanese Pharmacopoeia and the like.

In Japan, a preparation containing sodium N,2'-O-dibutyryladenosine-3',5'-phosphate is marketed under the name of Actosin (registered trademark) ointment; a preparation containing a recombinant type basic fibroblast growth factor is marketed under the name of Fiblast (registered trademark) spray; a preparation containing [(1-benzyl-1H-indazol-3-yl)oxy]acetic acid is marketed under the name of Zildasac (registered trademark) ointment; a preparation containing (±)-(2E,4E,6E,8E)-3,7-dimethyl-9-(2,6,6-trimethyl-1-cyclohexen-1-yl)-2,4,6,8-nonatetraenoic acid 3,4-dihydro-2,5,7,8-tetramethyl-2-(4,8,12-trimethyltridecyl)-2H-1-benzopyran-6-yl ester is marketed under the name of Olcenon (registered trademark) ointment; a preparation containing iodine is marketed under the name of Cadex (registered trademark) ointment; and a preparation containing povidone-iodine is marketed under the name of U-Pasta (registered trademark) Kowa, as a pharmaceutical product, respectively. Thus, these commercially available products can also be directly used.

Examples of the steroid preparation for external use include preparations containing clobetasol propionate, diflorasone diacetate, betamethasone butyrate propionate, difluprednate, diflucortolone valerate, mometasone furoate, fluocinonide, hydrocortisone butyrate propionate, betamethasone dipropionate, amcinonide, dexamethasone propionate, betamethasone valerate, deprodone propionate, beclomethasone dipropionate, dexamethasone valerate, aclomethasone propionate, prednisolone valerate acetate, or hydrocortisone butyrate. Examples of the activated vitamin D₃-containing preparation for external use include preparations containing tacalcitol, calcipotriol or maxacalcitol.

According to the present invention, the phrase "the epidermis has been thickened" or "epidermis thickening action" means that the thickness of the epidermis upon completion of the treatment has increased by 5% or more, preferably 10% or more, and more preferably 15% or more, as compared with the thickness of the epidermis before the initiation of treatment.

The measurement of the epidermal thickness can be carried out by a known method. For example, a tissue is collected from a vitiligo-affected site using a punch for biopsy, and the tissue is subjected sequentially to conventional formalin fixation and paraffin embedding. The thickness of the epidermis is measured at 5 to 10 arbitrarily selected sites per specimen, and the average value is taken as the thickness of the epidermis.

The amount of incorporation of the active pharmaceutical ingredient in the therapeutic agent may vary with the dosage form, but the amount is 0.001 to 30% by weight, and preferably 0.01 to 20% by weight, in a preparation for external use such as an ointment. In regard to the amount of administration of the therapeutic agent for vitiligo of the present invention, an appropriate amount of the therapeutic agent may be applied on the diseased site one to several times per day, in accordance with the size or severity of vitiligo. Concerning an injectable preparation or oral preparation, the amount of administration is not particularly limited as long as it is a therapeutically effective amount, but is 0.001 to 1000 mg, and preferably 0.01 to 100 mg, per day.

The therapeutic agent for vitiligo of the present invention can be used alone, or plural preparations can also be used concurrently. Furthermore, the therapeutic agent can be used in combination with a medicament such as a steroid preparation for external use or an activated vitamin D₃-containing preparation for external use, or with a phototherapy such as a PUVA therapy or a narrow-band UVB therapy.

### EXAMPLES

Hereinafter, the present invention will be explained in more detail by way of examples, but the present invention is not intended to be limited to these.

As test preparations, a (±)-(2E,4E,6E,8E)-3,7-dimethyl-9-(2,6,6-trimethyl-1-cyclohexen-1-yl)-2,4,6,8-nonatetraenoic acid 3,4-dihydro-2,5,7,8-tetramethyl-2-(4,8,12-trimethyltridecyl)-2H-1-benzopyran-6-yl ester-containing ointment (test preparation 1), a [(1-benzyl-1H-indazol-3-yl)oxy]acetic acid-containing ointment (test preparation 2), and a sodium N,2'-O-dibutyryladenosine-3',5'-phosphate-containing ointment (test preparation 3) were used.

### [Example 1] Measurement of skin color

Four-week old colored guinea pigs (body weight at the time of arrival: 200 to 400 g) which had been acclimated for one week, were shaved at the dorsal part with an electrical hair clipper and an electrical razor, to a size of about 6 cm x 6 cm. Furthermore, in order to apply the test preparations evenly, shaving was performed at a frequency of two times per week. Administration sites were prepared away from the midline, one each on the right and left sides (left: test preparation-applied part, right: non-treated part), at a size of 2 cm x 2 cm. The test preparations were applied once a day, in an amount of 30 mg, for 14 days. From the second day and thereafter, the application sites were cleaned by wiping with absorbent cotton wetted with lukewarm water, before applying the test preparations.

The L value (Lightness value) was measured as an index for the skin color, using a colorimeter. The results are presented in Table 1. As the color became darker, the L value was decreased. Thus, for all of the test preparations, it can be seen that the value significantly decreased as compared with the non-treated group.

A photograph of the dorsal part of the colored guinea pig obtained after the test preparation 1 was applied for 14 days, is presented in FIG. 1.

### [Example 2] Measurement of epidermal thickness

On the 15^{th} day after the initiation of the test, the colored guinea pigs were killed by exsanguination under ether anesthesia. The skins at the test preparation-applied part and the non-treated part were extracted and were fixed in a 10% neutrally buffered formalin solution, and were processed into paraffin sections. The sections were subjected to hematoxylin-eosin staining (HE staining), and the epidermal thicknesses were measured.

The measurement of the epidermal thickness was carried out such that the thickness of the epidermis was measured at five arbitrarily selected sites per specimen, and the average value was taken as the thickness of the epidermis.

The results are presented in Table 1. It can be seen that all of the test preparations have led to significant thickening of the epidermis as compared with the non-treated group.

As shown from the results of the skin color (Example 1) and the epidermal thickness (Example 2), in all cases of the test preparations, when the epidermal thickness increases, the value of the skin color is decreased. Therefore, it can be seen that pigmentation is accelerated as the epidermis is thickened.

### [Table 1]

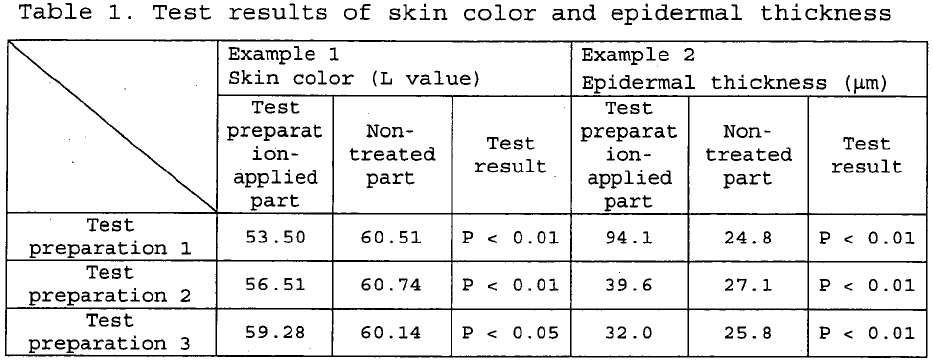

## Claims

1. A therapeutic agent for the treatment of vitiligo or for pigmentation which comprises a compound having an epidermis thickening action as an active pharmaceutical ingredient,
wherein the compound is selected from the group consisting of:
(a) (±)-(2E,4E,6E,8E)-3,7-dimethyl-9-(2,6,6-trimethyl-1-cyclohexen-1-yl)-2,4,6,8-nonatetraenoic acid 3,4-dihydro-2,5,7,8-tetramethyl-2-(4,8,12-trimethyltridecyl)-2H-1-benzopyran-6-yl ester, iodine, or povidone-iodine, or a pharmacologically acceptable salt thereof;
(b) a compound represented by the formula (II): 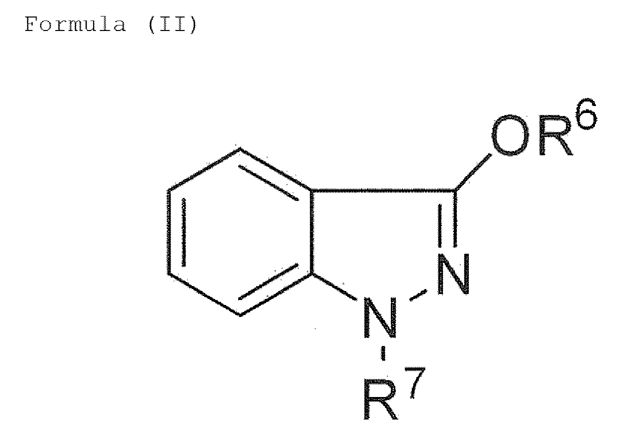 wherein R⁶ and R⁷, which may be identical or different, each represent a hydrogen atom, lower alkyl, carbonyl-lower alkyl, or substituted or unsubstituted aryl-lower alkyl, or a pharmacologically acceptable salt thereof;
(c) a compound represented by the formula (I): 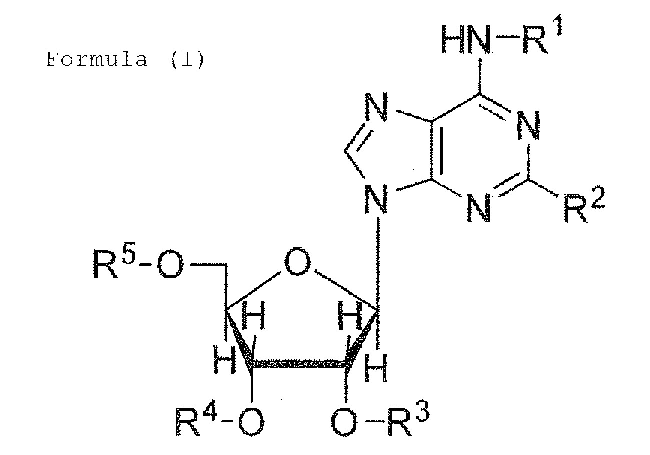 wherein R¹, R² and R³, which may be identical or different, are each a hydrogen atom, substituted or unsubstituted lower alkoxy, or substituted or unsubstituted lower alkanoyl; and R⁴ and R⁵, which may be identical or different, are each a hydrogen atom, or substituted or unsubstituted lower alkoxy, or R⁴ and R⁵ may be joined together to form a phosphoric acid ester, or a pharmacologically acceptable salt thereof;
(d) an acidic fibroblast growth factor, a basic fibroblast growth factor, an epidermal growth factor, a vascular endothelial growth factor, a keratinocyte growth factor and a platelet-derived growth factor.

2. The therapeutic agent according to claim 1, wherein the compound is selected from the group consisting of
(a) (±)-(2E,4E,6E,8E)-3,7-dimethyl-9-(2,6,6-trimethyl-1-cyclohexen-1-yl)-2,4,6,8-nonatetraenoic acid 3,4-dihydro-2,5,7,8-tetramethyl-2-(4,8,12-trimethyltridecyl)-2H-1-benzopyran-6-yl ester, iodine, or povidone-iodine, or a pharmacologically acceptable salt thereof;
(b') [(1-benzyl-1H-indazol-3-yl)oxy]acetic acid or a pharmacologically acceptable salt thereof;
(c') N,2'-O-dibutyryladenosine-3',5'-phosphoric acid, 2-[2-(4-chlorophenyl)ethoxy]adenosine, or a pharmacologically acceptable salt thereof:
(d') a recombinant type basic fibroblast growth factor.

3. The therapeutic agent according to claim 1, wherein the compound is selected from the group consisting of the said (a) and (b).

4. The therapeutic agent according to claim 2, wherein the compound is selected from the group consisting of the said (a) and (b').

5. The therapeutic agent according to claim 1 or 2, wherein the compound is selected from the group consisting of
(±)-(2E,4E,6E,8E)-3,7-dimethyl-9-(2,6,6-trimethyl-1-cyclohexen-1-yl)-2,4,6,8-nonatetraenoic acid 3,4-dihydro-2,5,7,8-tetramethyl-2-(4,8,12-trimethyltridecyl)-2H-1-benzopyran-6-yl ester or a pharmacologically acceptable salt thereof;
[(1-benzyl-1H-indazol-3-yl)oxy]acetic acid or a pharmacologically acceptable salt thereof; and N,2'-O-dibutyryladenosine-3',5'-phosphoric acid or a pharmacologically acceptable salt thereof.

6. Use of a compound for the production of a medicament for the treatment of vitiligo or for pigmentation, wherein the compound is selected from the group consisting of:
(a) (±)-(2E,4E,6E,8E)-3,7-dimethyl-9-(2,6,6-trimethyl-1-cyclohexen-1-yl)-2,4,6,8-nonatetraenoic acid 3,4-dihydro-2,5,7,8-tetramethyl-2-(4,8,12-trimethyltridecyl)-2H-1-benzopyran-6-yl ester, iodine, or povidone-iodine, or a pharmacologically acceptable salt thereof;
(b) a compound represented by the formula (II): 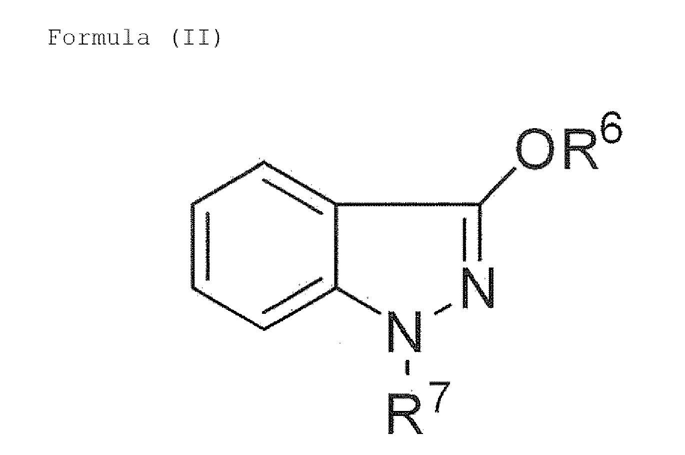 wherein R⁶ and R⁷, which may be identical or different, each represent a hydrogen atom, lower alkyl, carbonyl-lower alkyl, or substituted or unsubstituted aryl-lower alkyl, or a pharmacologically acceptable salt thereof;
(c) a compound represented by the formula (I): 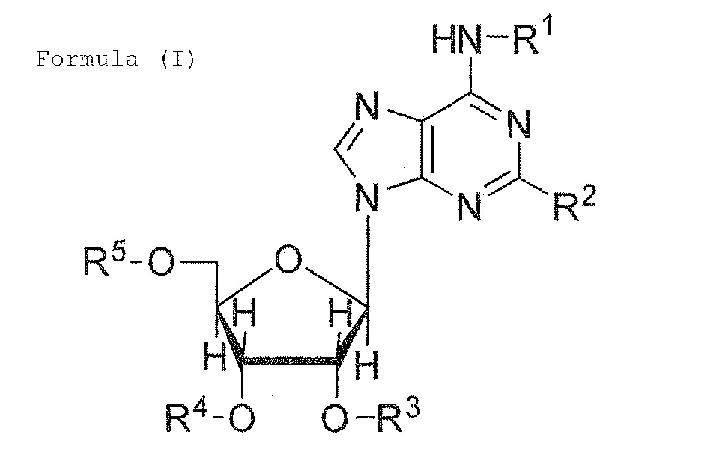 wherein R¹, R² and R³, which may be identical or different, are each a hydrogen atom, substituted or unsubstituted lower alkoxy, or substituted or unsubstituted lower alkanoyl; and R⁴ and R⁵, which may be identical or different, are each a hydrogen atom, or substituted or unsubstituted lower alkoxy, or R⁴ and R⁵ may be joined together to form a phosphoric acid ester, or a pharmacologically acceptable salt thereof;
(d) an acidic fibroblast growth factor, a basic fibroblast growth factor, an epidermal growth factor, a vascular endothelial growth factor, a keratinocyte growth factor and a platelet-derived growth factor.

7. The use according to claim 6, wherein the compound is selected from the group consisting of
(a) (±)-(2E,4E,6E,8E)-3,7-dimethyl-9-(2,6,6-trimethyl-1-cyclohexen-1-yl)-2,4,6,8-nonatetraenoic acid 3,4-dihydro-2,5,7,8-tetramethyl-2-(4,8,12-trimethyltridecyl)-2H-1-benzopyran-6-yl ester, iodine, or povidone-iodine, or a pharmacologically acceptable salt thereof;
(b') [(1-benzyl-1H-indazol-3-yl)oxy]acetic acid or a pharmacologically acceptable salt thereof;
(c') N,2'-O-dibutyryladenosine-3',5'-phosphoric acid, 2-[2-(4-chlorophenyl)ethoxy]adenosine, or a pharmacologically acceptable salt thereof:
(d') a recombinant type basic fibroblast growth factor.

8. The use according to claim 6, wherein the compound is selected from the group consisting of the said (a) and (b).

9. The use according to claim 7, wherein the compound is selected from the group consisting of the said (a) and (b').

10. The use according to claim 6 or 7, wherein the compound is selected from the group consisting of (±)-(2E,4E,6E,8E)-3,7-dimethyl-9-(2,6,6-trimethyl-1-cyclohexen-1-yl)-2,4,6,8-nonatetraenoic acid 3,4-dihydro-2,5,7,8-tetramethyl-2-(4,8,12-trimethyltridecyl)-2H-1-benzopyran-6-yl ester or a pharmacologically acceptable salt thereof;
[(1-benzyl-1H-indazol-3-yl)oxy]acetic acid or a pharmacologically acceptable salt thereof; and N,2'-O-dibutyryladenosine-3',5'-phosphoric acid or a pharmacologically acceptable salt thereof.
